# EUROPÄISCHE PATENTANMELDUNG

(11) **EP 1 413 970 A1**
(43) Veröffentlichungstag der Anmeldung: **28.04.2004**
(21) Anmeldenummer: 03405754.7
(22) Anmeldetag: 18.10.2003
(51) Int. Cl.: G06K 7/00

(54) **Verfahren zur Durchführung von Transaktionen im Krankenhausbereich mittels tragbaren Speichermodulen sowie Vorrichtung mit tragbaren Speichermodulen für den Krankenhausbereich**

(30) Priorität: 21.10.2002 CH 17632002
(71) Anmelder: Transrex AG, 6023 Rothenburg (CH)
(72) Erfinder: Frutiger, Heinz, 8915 Hausen am Albis (CH); Ganyi, Tibor, 6330 Cham (CH); Rychen, Christoph, 6274 Eschenbach (CH)
(74) Vertreter: Rutz, Peter

(57) **Zusammenfassung**

Das im Krankenhausbereich anzuwendende Verfahren ist zur Durchführung von dem Bezug von Waren und/oder Dienstleistungen dienenden Transaktionen geeignet. Dazu sind tragbare Speichermodule (3), insbesondere Speicherkarten, wie Chip Cards oder Smart Cards, vorgesehen, mittels denen Daten zu einem Lesegerät (12) einer Endvorrichtung (1, 2, 2') übertragbar sind, welche der Bestellung und/oder der Abgabe der Waren und/oder Dienstleistungen dient. Erfindungsgemäss ist die Endvorrichtung (1, 2, 2') über ein Netzwerk (100) mit einer Zentralvorrichtung (50) verbunden, welche die Transaktionen registriert und/oder steuert.

## Beschreibung

Die Erfindung betrifft ein Verfahren zur Durchführung von Transaktionen im Krankenhausbereich mittels tragbaren Speichermodulen sowie eine Vorrichtung mit tragbaren Speichermodulen für den Krankenhausbereich nach dem Oberbegriff des Patentanspruchs 1 bzw. 11.

Die Erfindung betrifft insbesondere ein Verfahren zur Durchführung von Transaktionen, mittels denen ein Patient Waren und/oder Dienstleistungen vom Krankenhausbetreiber beziehen kann.

Die nachstehend verwendeten Begriffe "Krankenhaus", "Krankenhausbereich" schliessen Gesundheits- und Betagteninstitute mit ein, in denen Personen betreut und/oder gepflegt werden.

Zur Bezahlung von Waren und Dienstleistungen werden im Krankenhausbereich oft tragbare Speichermodule, insbesondere mit einem Speicher versehene Karten, sogenannte Chip Cards oder Smart Cards, verwendet (siehe z.B. [1], US Patent 6,259,035 B1). Auf diese Karten können Patienten und Krankenhausangestellte einen Kredit übertragen lassen, der danach bei jeder Konsumation entsprechend reduziert wird. Ein Krankenhauspatient kann anhand der Karte beispielsweise Gebühren für Telefongespräche, Audio-, Video- und Fernsehübertragungen bezahlen, die zu einem am Krankenhausbett vorgesehenen Endsystem übertragen werden. Möglich ist ferner der Bezug von Waren in zentralen Restaurationsbetrieben, die den Karteninhabern zugänglich sind.

Die Transaktionen erfolgend dabei im off-line Betrieb, nach Einschub der Karte in den Kartenleser (siehe [2], US Patent 6,402,032 B1) eines den Karteninhabern zugänglichen Automaten oder eines einer Patienten zugeordneten Endvorrichtung.

Sofern genügend Kredit auf der eingeführten Karte registriert ist, kann der Patient die von ihm ausgewählten Waren und Dienstleistungen beziehen. Im Krankenhausbereich ist jedoch von besonderer Bedeutung, dass die Konsumationen der Patienten nicht im Widerspruch zu ärztlich verordneten Ernährungsplänen stehen. Dazu ist eine genaue Instruktion des Patienten erforderlich. Gelegentlich ist zu prüfen, welche Konsumationen getätigt wurden, was die Rücksprache mit Patienten erforderlich macht, welche oft nur unpräzise Angaben machen können.

Ferner werden die Hauptspeisen unter Berücksichtigung der individuellen Bedürfnisse normalerweise zentral vorbereitet, weshalb für das Personal hohe Aufwendungen und die Patienten oft unerwünschte Einschränkungen bezüglich der Auswahl der Menüauswahl sowie der Konsumationszeiten entstehen.

Der vorliegenden Erfindung liegt die Aufgabe zugrunde, ein verbessertes Verfahren zur Durchführung von Transaktionen im Krankenhausbereich mittels tragbaren Speichermodulen sowie eine entsprechende zur Kommunikation mit den tragbaren Speichermodulen geeignete Vorrichtung anzugeben.

Diese Aufgabe wird mit einem Verfahren und einer Vorrichtung gelöst, welche die in Anspruch 1 bzw. 11 angegebenen Merkmale aufweisen. Vorteilhafte Ausgestaltungen der Erfindung sind in weiteren Ansprüchen angegeben.

Das im Krankenhausbereich anzuwendende Verfahren ist zur Durchführung von dem Bezug von Waren und/oder Dienstleistungen dienenden Transaktionen geeignet. Dazu sind tragbare Speichermodule, insbesondere Speicherkarten, wie Chip Cards oder Smart Cards, vorgesehen, mittels denen Daten zu einem Lesegerät einer Endvorrichtung übertragbar sind, welche der Bestellung und/oder der Abgabe der Waren und/oder Dienstleistungen dient.

Erfindungsgemäss ist die Endvorrichtung über ein Netzwerk mit einer Zentralvorrichtung verbunden, welche die Transaktionen registriert und/oder steuert.

Anstelle des bisher üblichen off-line-Betriebs, während dessen lediglich die den Leistungsbezügen entsprechenden Belastungen auf das Speichermodul übertragen werden, wird erfindungsgemäss die Zentralvorrichtung an den Transaktionen beteiligt. Daraus resultieren vielfältige Vorteile, die nachstehend näher erläutert werden.

Die mittels der tragbaren Speichermodule durchgeführten Transaktionen erfolgend dabei zumeist im on-line-Betrieb, in dem die Zentralvorrichtung die Transaktionen registriert und/oder steuert. Möglich ist ferner, dass das Speichermodul zusätzlich zum Bezug von Waren und/oder Dienstleistungen an einer im off-line Betrieb arbeitenden Endvorrichtung, beispielsweise einem dezentral positionierten Kaffeeautomaten, geeignet ist, wodurch entsprechende Nutzungseinschränkungen vermieden werden.

Beim nachstehend beschriebenen on-line-Betrieb wird während einer Transaktion ein dem Anwender zugeordneter individueller Code zur Zentralvorrichtung übertragen, welche die Transaktion anhand von dem Anwender zugeordneten Datensätzen steuert. Die Zentralvorrichtung ist daher in der Lage, die Transaktion anwenderspezifisch zu steuern und den Anwender bei der Auswahl der Waren und/oder Dienstleistungen zu unterstützen bzw. die Auswahl der Waren und/oder Dienstleistungen entsprechend einem gegebenenfalls ärztlich verordneten Heilungs- oder Ernährungsprogramm entsprechen zu beschränken. In der Kantine sind für einen bestimmten Anwender, der im Krankenhaus als Patient registriert ist, beispielsweise nur fleischlose und weitgehend fettfreie Speisen wählbar. An einem Getränkeautomaten sind für diesen Patienten Kaffee und Zucker enthaltende Getränke gesperrt. Hinsichtlich des Bezugs von Dienstleistungen kann in einem Fitnessraum die Benutzung bestimmter Einrichtungen, z.B. Solarien, beschränkt sein. Ferner kann der Bezug von Video- oder TV- oder Telekommunikationsübertragungen beschränkt werden, für die in einer ersten dem Anwender zur alleinigen Nutzung zugeordneten, normalerweise am Krankenbett positionierten Endvorrichtung die entsprechenden Endgeräte, wie Kanalwähler, Bildschirm, Schallwandler, Telekommunikationsterminals, Computer, etc. angeordnet sind.

Die Zentralvorrichtung erzeugt basierend auf der Identität des Anwenders Filtersätze, mittels denen entsprechende Nutzungsoder Auswahlbeschränkungen in der benutzten Endvorrichtung vorgenommen werden.

Die Filtersätze werden vorzugsweise unter Berücksichtigung der Tageszeit und/oder der bereits registrierten Bezüge von Waren und/oder Dienstleistungen gebildet.

So kann beispielsweise der Bezug von Kaffe nach einer bestimmten Tageszeit gesperrt werden. Für jüngere Anwender kann beispielsweise der Bezug einzelner Videoübertragungen generell oder in Abhängigkeit der Tageszeit gesperrt werden.

Die Auswahl der Waren und/oder Dienstleistungen wird durch die beschränkte Auswahl erleichtert und kann somit schneller durchgeführt werden.

Für die Menüauswahl werden dem Anwender vorzugsweise nur die nicht beschränkten Speisen auf einem berührungsempfindlichen Bildschirm (Touch Screen) angezeigt, so dass die Durchführung der Transaktion weiter erleichtert wird.

Die Auswahl und/oder der Bezug von Waren und/oder Dienstleistungen kann dabei an der ersten dem Anwender individuell oder an einer zweiten, allen Anwendern oder einzelnen Anwendergruppen zugeordneten Endvorrichtung erfolgen.

Die Auswahl von Speisen an der ersten dem Anwender individuell zugeordneten Endvorrichtung, unter Berücksichtigung allfälliger Bezugsbeschränkungen, erleichtert die Logistik, da die Patienten, gegebenenfalls mit Unterstützung des Pflegepersonals, rasch ihre Auswahl für den folgenden Tag oder die gesamte Woche treffen können. Ein Verteilen von Fragebogen oder die individuelle Abfrage aller Patienten und die anschliessende Konsolidierung der Abfrageergebnisse entfallen. Diese problemlos ermittelten Daten erlauben insbesondere eine verbesserte Planung der Einkäufe der Krankenhausküche.

Bei der zweiten, allen Anwendern oder einzelnen Anwendergruppen zugeordneten Endvorrichtung, die beispielsweise in der Kantine des Krankenhauses installiert ist, kann die Auswahl der Speisen, nach Einschub des Speichermoduls, in gleicher Weise erfolgen. Nach Zusammenstellung eines Menüs generiert die zweite Endvorrichtung eine Bestellung für die ausgewählten Speisen. Die gegebenenfalls mit Datum, Uhrzeit, Anwenderposition und/oder Anwenderidentität versehene Bestellung wird beispielsweise ausgedruckt und/oder elektronisch an die Ausgabestelle übermittelt. Der Anwender trifft daher seine Auswahl, nimmt nach erfolgter Transaktion den gedruckten Bestellschein und holt die bestellten Speisen ab. Sofern die Bestellung elektronisch übermittelt wird, können die bestellten Speisen, wie bei den an das Krankenhausbett gebundenen Patienten auch ausgeliefert werden.

Vorzugsweise sind die Endvorrichtungen auch zur Ausgabe von Sprachsignalen geeignet, so dass die Anwender bei der Auswahl der Waren und/oder Dienstleistungen und bei der Durchführung der Transaktionen durch die Zentralvorrichtung und/oder die betreffende Endvorrichtung akustisch unterstützt werden können.

Die zur Durchführung der Transaktion genutzte zweite Endvorrichtung, beispielsweise ein Kaffeeautomat, kann nach erfolgter Transaktion die Waren und/oder Dienstleistungen auch direkt an den Anwender abgeben.

Basierend auf einer registrierten Bestellung können die erste oder zweite Endvorrichtung oder die Zentralvorrichtung auch weitere der Abgabe der Waren und/oder Dienstleistungen dienende Einrichtungen steuern. Beispielsweise werden Trainingsgeräte oder Solarien zur Nutzung freigegeben.

Die Registrierung des Bezugs von Waren und Dienstleistungen dient ferner dem optimierten Einkauf bzw. der Lagerbewirtschaftung und/oder der Rechnungsführung des Krankenhauses. Die Registrierung der Transaktionen kann ferner in vorteilhafter Weise zur Überwachung des Bezugs ärztlich verordneter Waren, Esswaren oder Medikamente, oder Dienstleistungen verwendet werden.

Für bestimmte Patientengruppen kann die Medikamentenabgabe nämlich mittels der erfindungsgemässen Vorrichtung erfolgen. In der Zentralvorrichtung oder einem damit verbundenen System, beispielsweise einem Abteilungsrechner, kann der erforderliche Medikamentenbezug eines Patienten registriert sein. Mittels des Speichermoduls kann der Patient seine Medikamente an einer gegebenenfalls automatisierten Ausgabestelle beziehen, wobei durch die Zentralvorrichtung oder damit verbundene Systeme mittels den registrierten Transaktionen überwacht wird, dass der Patient die verordneten Medikamente bezogen hat, jedoch nicht doppelt bezieht.

Mittels der Zentralvorrichtung kann ferner die Kostenbelastung gesteuert werden. Anhand der Anwenderidentität erfolgt beispielsweise eine Zuordnung zu einer Benutzergruppe, für die verschiedene Tarife festgelegt sind. Der Anwender kann beispielsweise einer der Benutzergruppe "PATIENT", "ARZT- UND PFLEGEPERSONAL", "VERWALTUNG" oder "BESUCHER" zugeordnet sein.

Die erfindungsgemässe Vorrichtung bzw. die Speichermodule können daher durch alle durch das Krankenhaus verwalteten Personen, Patienten und Personal, in vorteilhafter Weise benutzt werden.

Die Daten des Anwenders, welche die Zentralvorrichtung bei der Steuerung der Transaktionen hinzu zieht, sind in lokal oder dezentral, gegebenenfalls in den tragbaren Speichermodulen vorgesehenen Speicherbereichen abgelegt. Die Ablage der Daten kann daher zentral oder dezentral in den tragbaren Speichermodulen erfolgen, so dass diese auch für den off-line-Betrieb genutzt werden können.

Die Bezüge von Waren und Dienstleistungen, wenigstens deren Kosten, werden auf dem Speichermodul vorzugsweise geordnet nach Kategorie oder Kostenstellen registriert. Die Kosten für die Leistungsbezüge können auch mittels der Zentralvorrichtung registriert werden, so dass eine Doppelte Buchhaltung geführt wird.

In einer vorzugsweisen Ausgestaltung ist das Lesegerät der ersten und/oder der zweiten Endvorrichtung zum Lesen von Kreditkarten geeignet, so dass eine Kreditkartenbelastung zu Gunsten des Krankenhauses sowie eine entsprechende Gutschrift zu Gunsten des Anwenders bzw. Kreditkarteninhabers durch die Zentralvorrichtung registriert werden kann, welche eine entsprechende Gutschrift bei der nächsten Kontaktaufnahme an das tragbare Speichermodul überträgt. Diese erfindungsgemässen Massnahmen führen zu einer wesentlichen Vereinfachung des Zahlungsverkehrs.

Möglich ist ferner die Verwendung eines weiteren zur Aufnahme von Kreditkarten dienenden Lesegeräts, mittels dessen Kredite auf das Speichermodul übertragbar sind.

Ferner kann das tragbare Speichermodul auch zur Kontrolle des Zutritts des Anwenders zu Räumen oder Zonen des Krankenhausees verwendet werden.

Das Netzwerk, welches die Endvorrichtungen mit der Zentralvorrichtung verbindet, kann drahtgebunden oder drahtlos realisiert werden (siehe beispielsweise [3], Andrew S. Tanenbaum, Computernetzwerke, 3. Auflage, Prentice Hall 1998, Seiten 25-33). Die Datenübertragung von den tragbaren Speichermodulen zu den Endvorrichtungen kann ebenfalls drahtgebunden (mittels Kontaktierung der Speichereinheit) oder drahtlos (optisch, induktiv oder durch Übertragung elektromagnetischer Wellen) erfolgen.

Nachfolgend wird die Erfindung anhand von Zeichnungen näher erläutert. Dabei zeigt:
- Figur 1: eine erfindungsgemässe Vorrichtung mit ersten, Anwendern individuell zugeordneten Endvorrichtungen 1, mit zweiten allen Anwendern oder Gruppen von Anwendern zugeordneten Endvorrichtungen 2, mit einer Zentralvorrichtung 50, mit einer Gruppe 6 von Dienstleistungsvorrichtungen 61, ..., 64 sowie mit der Durchführung von Transaktionen dienenden Speichermodulen 3;
- Figur 2: eine erste Endvorrichtung 1 in einer vorzugsweisen Ausgestaltung;
- Figur 3: ein bevorzugtes Speichermodul 3 mit einem Chip 31 bzw. einer integrierten Schaltung, die mittels Kontakten 32 oder einer Induktionsschleife 33 mit einer ersten oder zweiten Endvorrichtung 1, 2 in Kontakt treten kann; und
- Figur 4: den Aufbau einer zweiten Endvorrichtung 2.

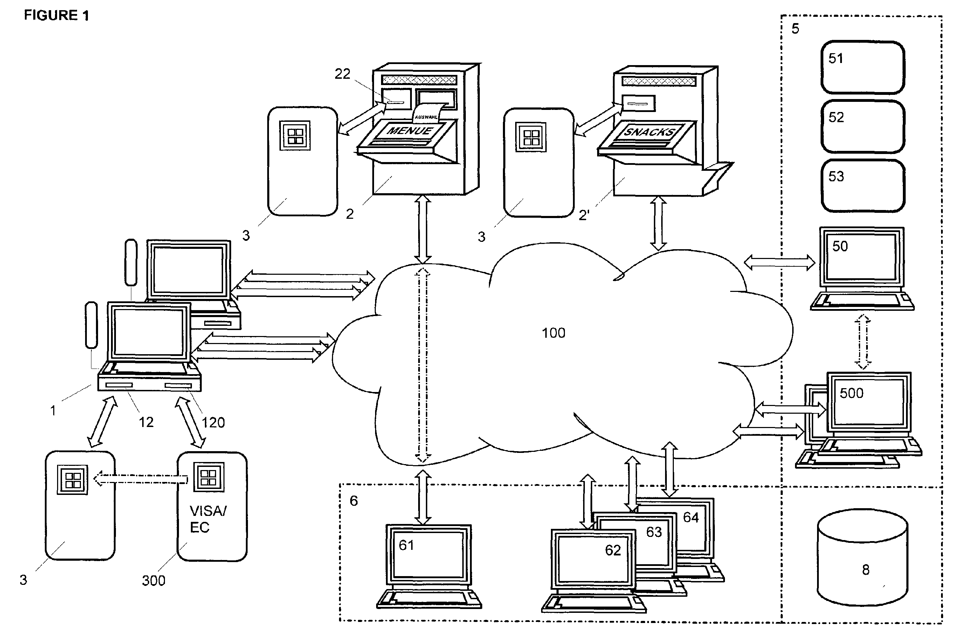

Figur 1 zeigt eine erfindungsgemässe Vorrichtung mit ersten, Anwendern individuell zugeordneten Endvorrichtungen 1, mit zweiten, allen Anwendern oder Gruppen von Anwendern zugeordneten Endvorrichtungen 2, die über ein Netzwerk 100 mit einer Zentralvorrichtung 50 verbunden sind. An das Netzwerk 100 ist ferner eine Gruppe 6 von Dienstleistungsvorrichtungen 61, ..., 64 sowie eine Gruppe mit der Zentralvorrichtung 50 verbundener Abteilungsrechner 500 angeschlossen. In der gezeigten Ausgestaltung der Erfindung ist die Gruppe 6 von Dienstleistungsvorrichtungen 61, ..., 64 und die Zentralvorrichtung 50 mit den Abteilungsrechnern 500, welche organisatorisch eine Verwaltungsgruppe 5 bilden, mit einer zentralen Datenbank 8 verbunden, in der die Daten der verwalteten Anwender, insbesondere der Krankenhauspatienten und des Personals abgelegt sind.

Gezeigt sind ferner die der Durchführung von Transaktionen dienenden Speichermodule 3, die in Lesevorrichtungen 12, 22 einschiebbar sind, die in der gezeigten Ausgestaltung in die ersten bzw. zweiten Endvorrichtungen 1, 2 integriert sind. Diese Transaktionen werden erfindungsgemäss von der Zentralvorrichtung registriert und/oder gesteuert.

Anstelle des bisher üblichen off-line-Betriebs, während dessen lediglich die den Leistungsbezügen entsprechenden Belastungen auf das Speichermodul 3 übertragen werden, wird erfindungsgemäss die Zentralvorrichtung an den Transaktionen beteiligt. Daraus resultieren vielfältige Vorteile, die oben erläutert wurden.

Die mittels der tragbaren Speichermodule 3 durchgeführten Transaktionen erfolgend dabei zumeist im on-line-Betrieb, in dem die Zentralvorrichtung die Transaktionen registriert und/oder steuert. Möglich ist ferner, dass das Speichermodul 3 zusätzlich zum Bezug von Waren und/oder Dienstleistungen an einer im off-line Betrieb arbeitenden Endvorrichtung, beispielsweise einem dezentral positionierten Kaffeeautomaten, geeignet ist, wodurch entsprechende Nutzungseinschränkungen vermieden werden.

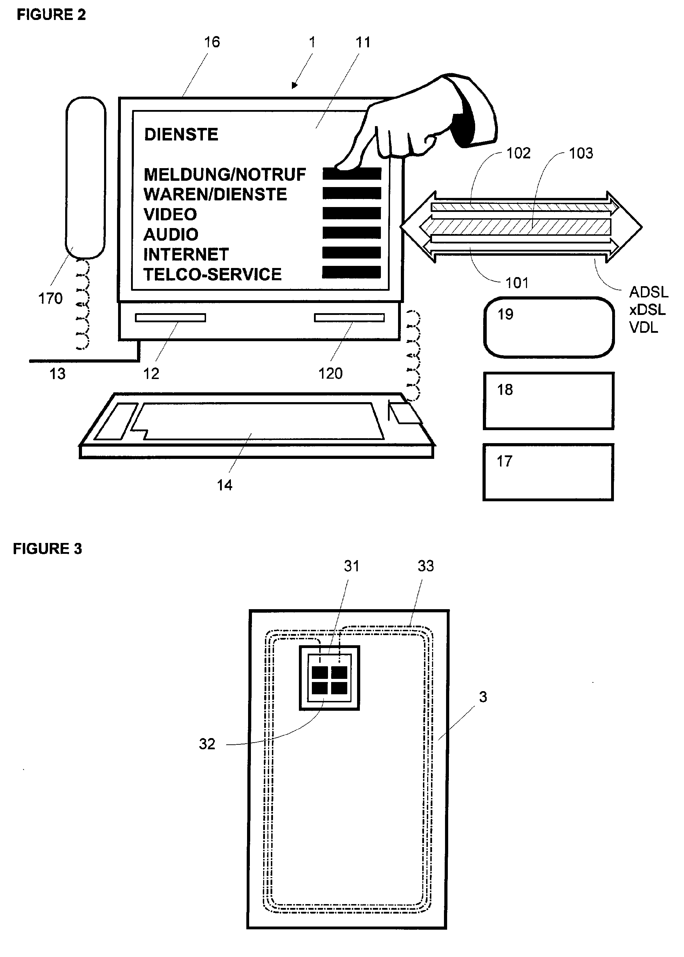

Von grosser Bedeutung sind die ersten, dem Anwender bzw. Krankenhauspatienten individuell zugeordneten Endvorrichtungen 1, von denen eine in Figur 2 in vorzugsweiser Ausgestaltung dargestellt ist.

Diese Endvorrichtung 1 umfasst wesentliche Elemente eines Personalcomputers, wie Zentralprozessor CPU, dynamischer und statischer Speicher RAM, ROM, gegebenenfalls eine Speicherplatte und ein CD oder DVD Laufwerk, Ein-/Ausgabemodule, insbesondere eine Netzwerkkarte, einen Bildschirm, vorzugsweise einen berührungsempfindlichen Bildschirm 11, sowie entsprechende Anwendungs- und Treiberprogramme und ein Betriebssystem, beispielsweise der MS-Windows Reihe. Die über ein Kabel 13 an das Netzwerk 100 anschliessbare Endvorrichtung 1 umfasst ferner ein Lesegerät 12 für die Speichermodule 3, ein Telefonendgerät mit einem Hörer oder einer Sprechgarnitur 170 und gegebenenfalls einen Kanalwähler für TV-, Videound/oder Audioprogramme.

Alle Komponenten der ersten Endvorrichtung 1 sind vorzugsweise in einem Gehäuse 16 integriert. Das Telefonendgerät und der mit dem TV-Netz verbundene Kanalwähler können extern aufgestellt werden, werden jedoch vorzugsweise als Telefonkarte 17 oder Tunerkarte 18 ebenfalls in das Gehäuse 16 der Endvorrichtung 1 integriert.

Ein Telefonhörer 15 und, falls erwünscht, eine Tastatur 14 können drahtlos (wireless, cordless), beispielsweise gemäss dem DECT- oder BLUETOOTH-Verfahren arbeitend, mit der Endvorrichtung verbunden sein (betreffend DECT siehe [4], B. Walke, Mobilfunknetze und ihre Protokolle, Band 2, Teubner Verlag Stuttgart 2000, Seiten 107-113; betreffend BLUETOOTH siehe beispielsweise [5], US Patent Application Nr. 2002/0077095 A1) .

Besonders wesentlich bei der ersten Endvorrichtung 1 ist ferner eine Steuervorrichtung 19 mittels der der Bezug von Waren und/oder Dienstleistungen steuerbar ist. Diese Steuervorrichtung kann als Softwaremodul oder als Hardwareeinheit in die Endvorrichtung integriert werden.

Die Vernetzung der Systeme erfolgt besonders vorteilhaft mittels der ADSL (Asymmetric-Digital-Subscriber-Line) Technologie. ADSL ist eine Technologie, welche den herkömmlichen analogen oder digitalen Telefonanschluss in einen Breitband-Internetzugang verwandelt. ADSL teilt die Übertragungskapazität des Kupferdrahts einer herkömmlichen Telefonleitung digital in drei unterschiedlich grosse Bereiche auf; zwei Kanäle 102, 103 für den Datentransport und einen Telefonkanal 101 für die Übertragung von Nutz- und Steuersignalen einer Telefonverbindung. Die Datenkanäle 102, 103 weisen dabei unterschiedliche Bandbreiten auf. Für den Datenkanal 103, der der Übertragung von Daten hin zur Endvorrichtung 1 dient (downstream), ist eine Übertragungskapazität von rund 5 Mbit/s vorgesehen. Für den Datenkanal 102, mit dem Daten vom Endgerät wegtransportiert werden (upstream), ist eine reduzierte Übertragungskapazität von rund 0,5 Mbit/s vorgesehen. Die ADSL-Technologie ist beispielsweise in [6], US Patent 6,445,712 B1 beschrieben.

Der Anschluss der Endvorrichtungen 1 an das Netzwerk 100 mittels ADSL Technologie erlaubt dem Anwender, gleichzeitig zu telefonieren und Daten über das Internet auszutauschen. Ferner kann eine TCP/IP Verbindung (siehe [3], Seiten 442-449 und Seiten 556-558) zur Zentralvorrichtung 50 aufgebaut werden, über die vorzugsweise basierend auf dem Hyper Text Transfer Protokoll HTTP Daten zwischen der Zentralvorrichtung 50 und der Endvorrichtung 1 übertragen werden (siehe [3], Seiten 722-743).

Parallel dazu kann ferner ein internes lokales Netzwerk vorhanden sein, das beispielsweise auf der ETHERNET-Technologie basiert, welche die Dienstleistungen der auf die Bitübertragungsschicht und die Sicherungsschicht aufgesetzten MAC-Teilschicht einer Netzwerkarchitektur zur Verfügung stellt (siehe [3], Seiten 303-309).

Der Zugang zum Internet und die Übertragung von Instruktionen, insbesondere Steuerbefehlen kann zudem über TCP/IP - Verbindungen erfolgen, die auf dem lokalen, gegebenenfalls nach Ethernettechnologie errichteten Netzwerk errichtet werden (siehe [7], A. Shaikh, K.J. Christensen, Traffic Characteristics of Bulk Data Transfer using TCP/IP over Gigabit Ethernet).

In Figur 1 sind ferner zweite Endvorrichtungen 2, 2' gezeigt, die anhand der Speichermodule 3 von allen Anwendern oder nur von bestimmten Anwendergruppen benutzt werden können.

Figur 3 zeigt ein bevorzugtes Speichermodul 3 in der Form einer Chipkarte mit einem Chip 31 bzw. einer integrierten Schaltung, die mittels Kontakten 32 oder einer Induktionsschleife 33 mit einer ersten oder zweiten Endvorrichtung 1, 2 in Kontakt treten kann.

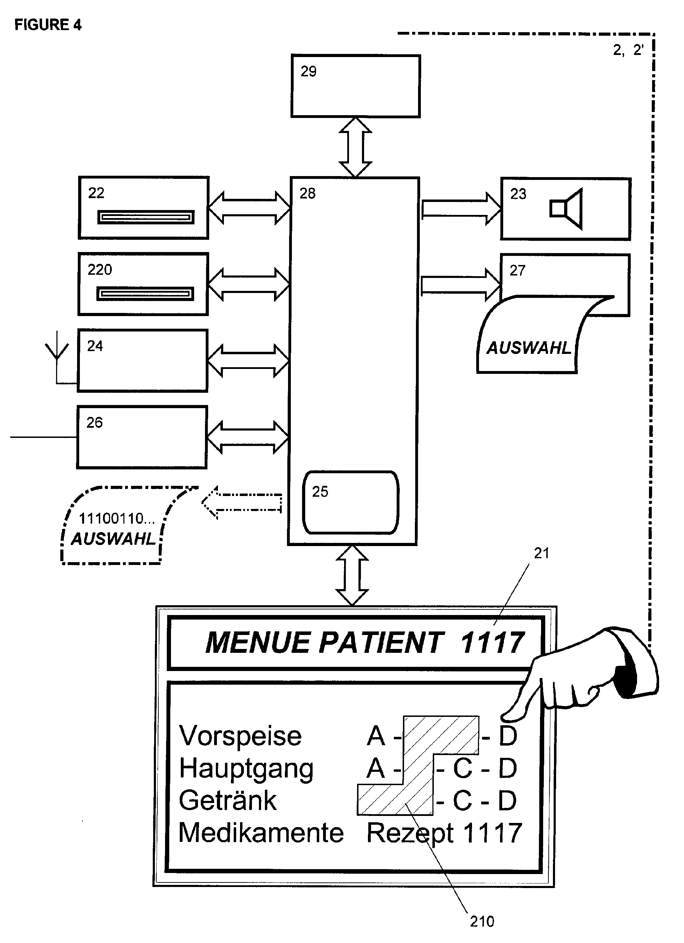

Figur 4 zeigt in einer vorzugsweisen Ausgestaltung den modularen Aufbau der zweiten Endvorrichtungen 2, 2' von Figur 1, von denen eine Endvorrichtung 2 lediglich zur Ausgabe oder elektronischen Weiterleitung einer mittels der Transaktion durchgeführten Bestellung und die andere Endvorrichtung 2' zur Ausgabe der ausgewählten Waren dient.

Die zweite Endvorrichtung 2, 2' weist einen Zentralprozessor 28, eine Speichereinheit 29, ein Lesegerät 22 für Speichermodule 3, eine Netzwerkkarte 26, ein erstes und ein zweites Ausgabemodul 25, 27 und einen berührungsempfindlichen Bildschirm (Touchscreen) auf, auf dem die Waren und/oder Dienstleistungen angezeigt werden und mittels Fingerdruck selektiert werden können.

Optional ist ferner ein Kreditkartenleser 220 und eine Audioeinheit 23 vorgesehen, mittels der der Anwender bei der Auswahl der Waren und/oder Dienstleistungen und bei der Durchführung der Transaktionen durch die Zentralvorrichtung 50 und/oder die betreffende zweite Endvorrichtung 2, 2' akustisch unterstützt werden kann.

Zum kontaktlosen Austausch von Daten zwischen dem Speichermodul 3 und der zweiten Endvorrichtung 2, 2' ist ferner ein Sende-/Empfänger (Transceiver) 24 vorgesehen der zum Empfang und zum Senden induktiver oder elektromagnetischer, gegebenenfalls optischer Signale geeignet ist.

Nach Durchführung einer Transaktion zur Auswahl von Warenund/oder Dienstleistungen erfolgt, entweder
a) gesteuert durch das erste Ausgabemodul 25, die Ausgabe von Waren unmittelbar bei der zweiten Endvorrichtung 2', oder
b) gesteuert durch das zweite Ausgabemodul 27, die Ausgabe eines Bestellscheines oder die elektronische Weiterleitung der Bestellung zu einer Ausgabestelle bzw. einer Dienstleistungsvorrichtung 61, ..., 64.

In Kantinen können beispielsweise zweite Endvorrichtungen 2 vorgesehen sein, die lediglich der Aufnahme und Ausgabe (Bestellschein) oder Weiterleitung einer Bestellung dienen (elektronische Bestellung).

Beim on-line-Betrieb der ersten und zweiten Endvorrichtungen 1, 2, 2' wird während einer Transaktion ein dem Anwender zugeordneter individueller Code zur Zentralvorrichtung 50 übertragen, welche die Transaktion anhand von dem Anwender zugeordneten Datensätzen steuert, die beispielsweise aus einer Datenbank 8 abrufbar sind. Die Zentralvorrichtung 50 ist daher in der Lage, die Transaktion anwenderspezifisch zu steuern und den Anwender bei der Auswahl der Waren und/oder Dienstleistungen zu unterstützen bzw. die Auswahl der Waren und/oder Dienstleistungen entsprechend einem gegebenenfalls ärztlich verordneten Heilungs- oder Ernährungsprogramm entsprechen zu beschränken.

Die Zentralvorrichtung erzeugt basierend auf der Identität des Anwenders Filtersätze, mittels denen entsprechende Nutzungsoder Auswahlbeschränkungen in der genutzten Endvorrichtung 1, 2, 2' vorgenommen werden.

Die Filtersätze werden vorzugsweise unter Berücksichtigung der Tageszeit und/oder der bereits registrierten Bezüge von Waren und/oder Dienstleistungen gebildet.

Für die Menüauswahl werden dem Anwender vorzugsweise nur die nicht beschränkten Speisen auf einem berührungsempfindlichen Bildschirm (Touch Screen) angezeigt. In Figur 4 ist dargestellt, dass verschiedene in einer Sperrzone 210 liegende Speisen nicht auswählbar sind. Die Darstellung der auswählbaren Speisen und/oder Dienstleistungen kann jedoch in beliebiger Form erfolgen.

Vorzugsweise sind die Endvorrichtungen auch zur Ausgabe von Sprachsignalen geeignet, so dass die Anwender bei der Auswahl der Waren und/oder Dienstleistungen und bei der Durchführung der Transaktionen durch die Zentralvorrichtung und/oder die betreffende Endvorrichtung akustisch unterstützt werden können.

In einer weiteren vorzugsweisen Ausgestaltung erfolgt die Steuerung der ersten und/oder zweiten Endvorrichtung 1, 2, 2' sprachgesteuert (siehe [5], ganzes Dokument), so dass körperlich behinderte Anwender die Endvorrichtungen 1, gegebenenfalls 2, 2' ebenfalls benutzen können.

Die Registrierung des Bezugs von Waren und Dienstleistungen dient ferner dem optimierten Einkauf bzw. der Lagerbewirtschaftung und/oder der Rechnungsführung des Krankenhauses. Dazu sind in der Zentralvorrichtung 50 entsprechende Module 51, 52 vorgesehen, welche die registrierten Transaktionen analysieren und entsprechende Finanzbuchungen oder Lagerbuchungen durchführen.

Die Registrierung der Transaktionen kann ferner in vorteilhafter Weise zur Überwachung des Bezugs ärztlich verordneter Waren, Esswaren oder Medikamente, oder Dienstleistungen verwendet werden.

Dazu ist in der Zentralvorrichtung oder in damit verbundenen Systemen 500, beispielsweise Abteilungsrechnern, ein Überwachungsmodul 53 vorgesehen, mittels dessen der Bezug von Waren- und/oder Dienstleistungen, gegebenenfalls der Bezug von Medikamenten oder Behandlungen überwacht und geprüft wird. Sofern die verordneten Leistungen innerhalb einer vorgesehen Zeitspanne nicht bezogen wurden, wird beispielsweise auf den Abteilungsrechnern 500 eine entsprechende Meldung ausgegeben.

Mittels der Zentralvorrichtung kann ferner die Kostenbelastung gesteuert werden.

Die Daten des Anwenders, welche die Zentralvorrichtung bei der Steuerung der Transaktionen hinzu zieht, können auch in den tragbaren Speichermodulen 3 abgelegt sein, so dass diese auch für den off-line-Betrieb genutzt werden können.

Die Bezüge von Waren und Dienstleistungen, wenigstens deren Kosten, werden auf dem Speichermodul 3 vorzugsweise geordnet nach Kategorie oder Kostenstellen registriert. Die Kosten für die Leistungsbezüge können auch mittels der Zentralvorrichtung 50 registriert werden, so dass eine Doppelte Buchhaltung geführt wird.

In einer vorzugsweisen Ausgestaltung ist das Lesegerät 12, 22 der ersten und/oder der zweiten Endvorrichtung zum Lesen von Kreditkarten geeignet, so dass eine Kreditkartenbelastung zu Gunsten des Krankenhauses sowie eine entsprechende Gutschrift zu Gunsten des Anwenders bzw. Kreditkarteninhabers durch die Zentralvorrichtung 50 registriert werden kann, welche eine entsprechende Gutschrift bei der nächsten Kontaktaufnahme an das tragbare Speichermodul 3 überträgt. Diese erfindungsgemässen Massnahmen führen zu einer wesentlichen Vereinfachung des Zahlungsverkehrs.

Möglich ist ferner die Verwendung eines weiteren zur Aufnahme von Kreditkarten dienenden Lesegeräts 120, 220 mittels dessen Kredite auf das Speichermodul übertragbar sind.

Ferner kann das tragbare Speichermodul 3 auch zur Kontrolle des Zutritts des Anwenders zu Räumen oder Zonen des Krankenhauses verwendet werden.

Die erfindungsgemässe Verbindungsvorrichtung wurde in einer bevorzugten Ausgestaltung beschrieben und dargestellt. Anhand der erfindungsgemässen Lehre sind jedoch weitere fachmännische Ausgestaltungen realisierbar.

### Literaturverzeichnis

[1] US Patent 6,259,035 B1
[2] US Patent 6,402,032 B1
[3] Andrew S. Tanenbaum, Computernetzwerke, 3. Auflage, Prentice Hall, München 1998
[4] B. Walke, Mobilfunknetze und ihre Protokolle, Band 2, Teubner Verlag Stuttgart 2000
[5] US Patent Application Nr. 2002/0077095 A1
[6] US Patent 6,445,712 B1
[7] A. Shaikh, K.J. Christensen, Traffic Characteristics of Bulk Data Transfer using TCP/IP over Gigabit Ethernet

## Patentansprüche

1. Verfahren zur Durchführung von dem Bezug von Waren und/oder Dienstleistungen dienenden Transaktionen im Krankenhausbereich, mittels tragbaren Speichermodulen (3), insbesondere Speicherkarten, wie Chip Cards oder Smart Cards, mittels denen Daten zu einem Lesegerät (12; 22) einer Endvorrichtung (1; 2; 2') übertragbar sind, welche der Bestellung und/oder der Abgabe der Waren und/oder Dienstleistungen dient, **dadurch gekennzeichnet, dass** die Endvorrichtung (1; 2; 2') über ein Netzwerk (100) mit einer Zentralvorrichtung (50) verbunden ist, welche die Transaktionen registriert und/oder steuert.

2. Verfahren nach Anspruch 1, **dadurch gekennzeichnet, dass** mittels den tragbaren Speichermodulen (3) Transaktionen im on-line-Betrieb, in dem die Zentralvorrichtung (50) die Transaktionen registriert und/oder steuert, oder im off-line-Betrieb, ohne Aktivierung der Zentralvorrichtung (50), durchgeführt werden.

3. Verfahren nach Anspruch 1 oder 2, **dadurch gekennzeichnet, dass** während einer Transaktion ein dem Anwender zugeordneter individueller Code zur Zentralvorrichtung (50) übertragen wird, welche die Transaktion anhand von dem Anwender zugeordneten Datensätzen steuert, die in lokal oder dezentral, gegebenenfalls in den tragbaren Speichermodulen (3) vorgesehenen Speicherbereichen (8) abgelegt sind, auf die die Zentralvorrichtung (50) zugreifen kann.

4. Verfahren nach Anspruch 3, **dadurch gekennzeichnet, dass** die Zentralvorrichtung (50) die Kostenbelastung und/oder das Angebot der Waren und/oder Dienstleistungen steuert, die mittels einer durchgeführten Transaktion an den Anwender abgegeben werden.

5. Verfahren nach Anspruch 4, **dadurch gekennzeichnet, dass** die Zentralvorrichtung (50), gegebenenfalls unter Berücksichtigung der Uhrzeit oder bereits erfolgtem Bezug von Waren oder Dienstleistungen, die dem Anwender nicht beziehungsweise nicht mehr zugänglichen Waren, wie Esswaren und Medikamente, und/oder Dienstleistungen, wie die Übertragung elektronischer Medien oder Körperbehandlungen, sperrt.

6. Verfahren nach einem der Ansprüche 1 bis 5, **dadurch gekennzeichnet, dass**
a) die zur Durchführung der Transaktion genutzte Endvorrichtung (1; 2) eine gegebenenfalls mit Datum, Uhrzeit, Anwenderposition und/oder Anwenderidentität versehene, gedruckte oder elektronische Bestellung für die ausgewählten Waren und/oder Dienstleistungen generiert, mittels der der Bezug oder die Ausgabe der Waren und/oder Dienstleistungen an den entsprechenden Ausgabestellen (6) erfolgen kann oder dass
b) die Endvorrichtung (2') die Waren und/oder Dienstleistungen direkt an den Anwender abgibt oder dass
c) die Zentralvorrichtung (50) oder die Endvorrichtung (1; 2) weitere der Abgabe der Waren und/oder Dienstleistungen dienende Einrichtungen (61; 62; 63) steuern.

7. Verfahren nach einem der Ansprüche 1 bis 6, **dadurch gekennzeichnet, dass** mittels der Registrierung des Bezugs von Waren und Dienstleistungen die Lagerbewirtschaftung und/oder zur Rechnungsführung des Krankenhauses erfolgt oder dass mittels der Registrierung der Transaktionen der Bezug ärztlich verordneter Waren, Esswaren oder Medikamente, oder Dienstleistungen überwacht wird.

8. Verfahren nach einem der Ansprüche 1 bis 7, **dadurch gekennzeichnet, dass** die Bezüge von Waren und Dienstleistungen, wenigstens deren Kosten, auf dem Speichermodul (3) vorzugsweise geordnet nach Kategorie oder Kostenstellen registriert werden.

9. Verfahren nach einem der Ansprüche 1 bis 8, **dadurch gekennzeichnet, dass** Kredite von einer Kreditkarte (300) auf das Speichermodul (3) übertragbar sind.

10. Verfahren nach einem der Ansprüche 1 bis 9, **dadurch gekennzeichnet, dass** mittels des Speichermoduls (3) der Zutritt des Anwenders zu Räumen oder Zonen des Krankenhauses kontrolliert wird.

11. Vorrichtung zur Durchführung von dem Bezug von Waren und/oder Dienstleistungen dienenden Transaktionen im Krankenhausbereich nach dem Verfahren gemäss einem der Ansprüche 1 bis 10, mittels tragbaren Speichermodulen (3), insbesondere Speicherkarten, wie Chip Cards oder Smart Cards, mittels denen Daten zu einem Lesegerät (12; 22) einer Endvorrichtung übertragbar sind, welches der Bestellung und/oder der Abgabe der Waren und/oder Dienstleistungen dient, **dadurch gekennzeichnet, dass** mehrere Endvorrichtungen (1; 2; 2') mittels eines Netzwerks (100) mit einer Zentralvorrichtung (50) verbunden sind, welche an den Endvorrichtungen (1; 2; 2') durchgeführte Transaktionen registriert und/oder steuert.

12. Vorrichtung nach Anspruch 11, **dadurch gekennzeichnet, dass** mittels den tragbaren Speichermodulen (3) Transaktionen im on-line-Betrieb, in dem die Zentralvorrichtung (50) die Transaktionen registriert und/oder steuert, oder im off-line-Betrieb, ohne Aktivierung der Zentralvorrichtung (50), durchführbar sind.

13. Vorrichtung nach Anspruch 11 oder 12, **dadurch gekennzeichnet, dass** während einer Transaktion ein dem Anwender zugeordneter individueller Code zur Zentralvorrichtung (50) übertragbar ist, welche die Transaktion anhand von dem Anwender zugeordneten Datensätzen steuert, die in lokal oder dezentral, gegebenenfalls in den tragbaren Speichermodulen (3) vorgesehenen Speicherbereichen (8) abgelegt und von der Zentralvorrichtung (50) abrufbar sind.

14. Vorrichtung nach Anspruch 13, **dadurch gekennzeichnet, dass** die Zentralvorrichtung (50) zur Steuerung der Kostenbelastung und/oder des Angebots der Waren und/oder Dienstleistungen geeignet ist, die mittels einer durchgeführten Transaktion an den Anwender abgegeben werden.

15. Vorrichtung nach Anspruch 14, **dadurch gekennzeichnet, dass** die Zentralvorrichtung (50), gegebenenfalls unter Berücksichtigung der Uhrzeit oder des bereits erfolgten Bezugs von Waren oder Dienstleistungen, zur Bildung eines an die Endvorrichtung (1; 2; 2') übertragbaren Filters (210) geeignet ist, mittels dessen die Ausgabe dem Anwender nicht beziehungsweise nicht mehr zugänglicher Waren, wie Esswaren und Medikamente, und/oder Dienstleistungen, wie die Übertragung elektronischer Medien oder Körperbehandlungen, sperrbar ist.

16. Vorrichtung nach einem der Ansprüche 11 bis 15, **dadurch gekennzeichnet, dass**
a) erste den Anwendern individuell zugeordnete Endvorrichtungen (1) vorgesehen sind, mittels denen ein Anwender Waren und/oder Dienstleistungen bestellen oder direkt beziehen kann;
b) zweite allen Anwendern oder Gruppen von Anwendern zugeordnete Endvorrichtungen (2; 2') vorgesehen sind, mittels denen Anwender Waren und/oder Dienstleistungen bestellen oder direkt beziehen können.

17. Vorrichtung nach Anspruch 16, **dadurch gekennzeichnet, dass** die die zur Durchführung der Transaktion genutzte erste oder zweite Endvorrichtung (1; 2) zur Erzeugung einer gegebenenfalls mit Datum, Uhrzeit, Anwenderposition und/oder Anwenderidentität versehenen elektronischen und/oder gedruckten Bestellung für die ausgewählten Waren und/oder Dienstleistungen geeignet ist, mittels der der Bezug oder die Ausgabe der Waren und/oder Dienstleistungen an den entsprechenden Ausgabestellen (6) erfolgen kann oder mittels der die Zentralvorrichtung (50) oder die erste oder zweite Endvorrichtung (1; 2) weitere der Abgabe der Waren und/oder Dienstleistungen dienende Einrichtungen (61; 62; 63) steuern.

18. Vorrichtung nach einem der Ansprüche 11 bis 17, **dadurch gekennzeichnet, dass** die Zentralvorrichtung (50) oder damit verbundene Systeme (500) der Lagerbewirtschaftung und/oder der Rechnungsführung dienende Module (51; 52) aufweisen, die zur Verarbeitung der registrierten Transaktionen geeignet sind oder dass die Zentralvorrichtung (50) oder die damit verbundenen, gegebenenfalls Anwendergruppen zugeordneten Systeme (500) mit einem Überwachungsmodul (53) versehen sind, mittels dessen der Bezug ärztlich verordneter Waren, Esswaren oder Medikamente, oder Dienstleistungen überwacht wird.

19. Vorrichtung nach einem der Ansprüche 11 bis 17, **dadurch gekennzeichnet, dass** das Lesegerät (12; 22) der ersten und/oder der zweiten Endvorrichtung (1; 2; 2') zum Lesen von Kreditkarten geeignet ist, oder dass die erste und/oder die zweite Endvorrichtung (1; 2; 2') mit einem weiteren Lesegerät (120; 220) mittels dessen Kredite auf das Speichermodul (3) übertragbar sind.

20. Vorrichtung nach einem der Ansprüche 11 bis 17, **dadurch gekennzeichnet, dass** die erste und oder die zweite Endvorrichtung (1; 2; 2') mit einem berührungsempfindlichen Bildschirm versehen ist.
